# EUROPEAN PATENT APPLICATION

(11) **EP 0 965 309 A1**
(43) Date of publication of application: **22.12.1999**
(21) Application number: 98111208.9
(22) Date of filing: 18.06.1998
(51) Int. Cl.: A61B 17/39

(54) **Bipolar electrosurgical scissors**

(71) Applicant: àWengen, Daniel F., 4106 Therwil (CH)
(72) Inventor: àWengen, Daniel F., 4106 Therwil (CH)
(74) Representative: Blum, Rudolf Emil Ernst

(57) **Abstract**

The bipolar electrosurgical scissors comprise two scissor members (1, 2), each of which is divided into a blade section (7, 8) and an electrode section (9, 10). The blade sections (7, 8) are used for cutting tissue while the electrode sections (9, 10) are used for cauterisation. The electrode sections (9, 10) form the distal end of the scissors and are electrically insulated from the blade sections (7, 8). Wires (17) are provided for supplying the cauterisation current. The scissors are easy to manufacture and convenient to handle.

## Description

The invention relates to bipolar electrosurgical scissors according to the preamble of claim 1.

Such scissors are used for cauterising and cutting tissues. For this purpose, they are designed to pass a high frequency current through the tissue, thereby causing hemostasis.

In known embodiments, the scissor blades are used as electrodes and an HF voltage can be applied between them. In order to prevent a short circuit between the blades while maintaining their shearing properties, special insulation techniques are required, such as they are e.g. described in US 5 700 261. Such insulations are, however, complicated an expensive.

In addition to this, handling known electrosurgical scissors properly is not easy because cutting and cauterisation are carried out by the same instrument and are therefore difficult to control.

The problem to be solved by the present invention is therefore to overcome at least part of these disadvantages. In particular, it is an object of the invention to provide bipolar electrosurgical scissors that are easy to use while being inexpensive in production. This problem is solved by the scissors according to claim 1.

By dividing the scissor members into a blade section and an electrode section, wherein the electrode section is arranged at a distal end of the blade section, a separation of the two functions is achieved that makes production as well as handling of the scissors easier.

Preferably, the electrode sections form a forceps for squeezing tissue between them, while the blade sections are used for cutting.

The surgeon can therefore first cauterise tissue with the distal end of his scissors and then cut it using the blade sections.

In a preferred embodiment, each electrode forms a cauterising surface substantially perpendicular to the direction of the scissors' pivotal movement. The cauterising surfaces can be arranged such that they are adjacent and facing each other when the scissors are closed.

In most applications, at least one of the electrode sections should be insulated from the corresponding blade section. Conductors can be arranged in the blade sections for carrying current to the electrodes. The conductors are insulated against the blade sections and preferably embedded therein.

Further embodiments and preferred features of the scissors according to the present invention can be found in the dependent claims as well as in the following description of a preferred embodiment with reference to the figures, wherein
Fig. 1 shows a complete view of a preferred embodiment of scissors according to the present invention,
Figs. 2 and 3 show the blade and electrode sections of the scissors in closed and opened positions,
Fig. 4 is a side view of the blade and electrode sections of Fig. 2,
Fig. 5 is a section along line V-V of Fig. 2, and
Fig. 6 is a section along line VI-VI of Fig. 2.

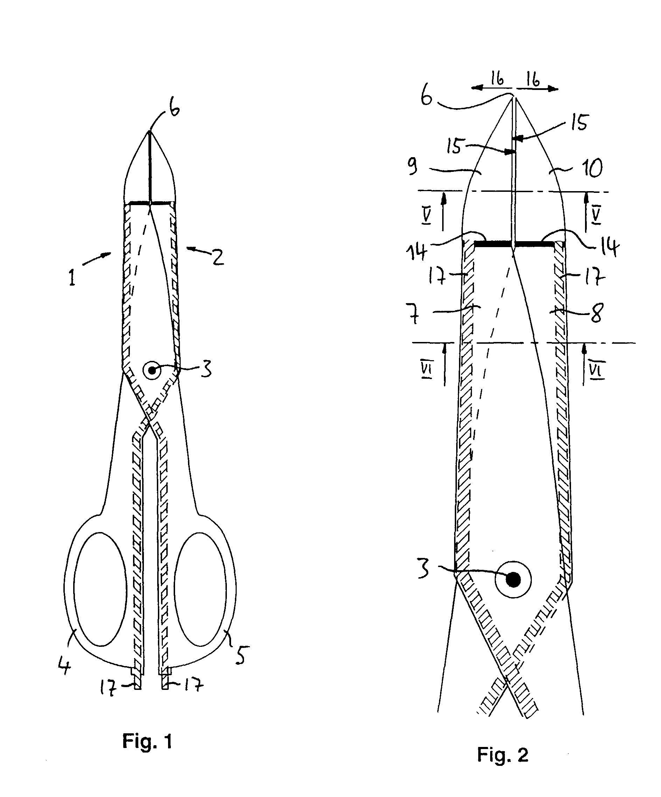

The basic design of a preferred embodiment of the invention is shown in Fig. 1. The scissors comprise two scissor members 1, 2 pivotally connected by a pivot pin 3. They can be operated conventionally by grip members 4, 5.

As can be seen in Figs. 2 - 4, each scissor member 1, 2 comprises a blade section 7, 8 and an electrode section 9, 10. The blade sections 7, 8 extend from pivot pin 3 to the electrode sections 9, 10 and the electrode sections 9, 10 from the blade sections 7, 8 to a distal tip 6.

The blade sections 7, 8 form a conventional shearing mechanism for severing tissue between cutting edges 11, 12. When viewing the closed scissors along the axis of pivot spindle 3 (cf. Fig. 2), the blade sections overlap partially. The blade sections are preferably made of a hard, stainless steel.

The electrode sections 9, 10 are arranged at a distal side (i.e. towards the scissors' tip 6) of the blade sections 7, 8 and form the distal end of the scissors. They are made of stainless steel and are electrically insulated from the blade sections 7, 8 by means of ceramic layers 14.

As can be seen from Figs. 2, 4 and 5, the electrode sections 9, 10 are, in the direction of the axis of pivot spindle 3, twice as thick as the blade sections 7, 8. They comprise two flat cauterising surfaces 15, which are perpendicular to the pivoting directions 16 of the scissor members 1, 2. When the scissors are closed, the cauterising surfaces 15 are adjacent to and facing each other and are extending in the scissors' longitudinal direction. In other words, the electrode sections 9, 10 are forming a forceps well suited for squeezing tissue between their cauterising surfaces 15.

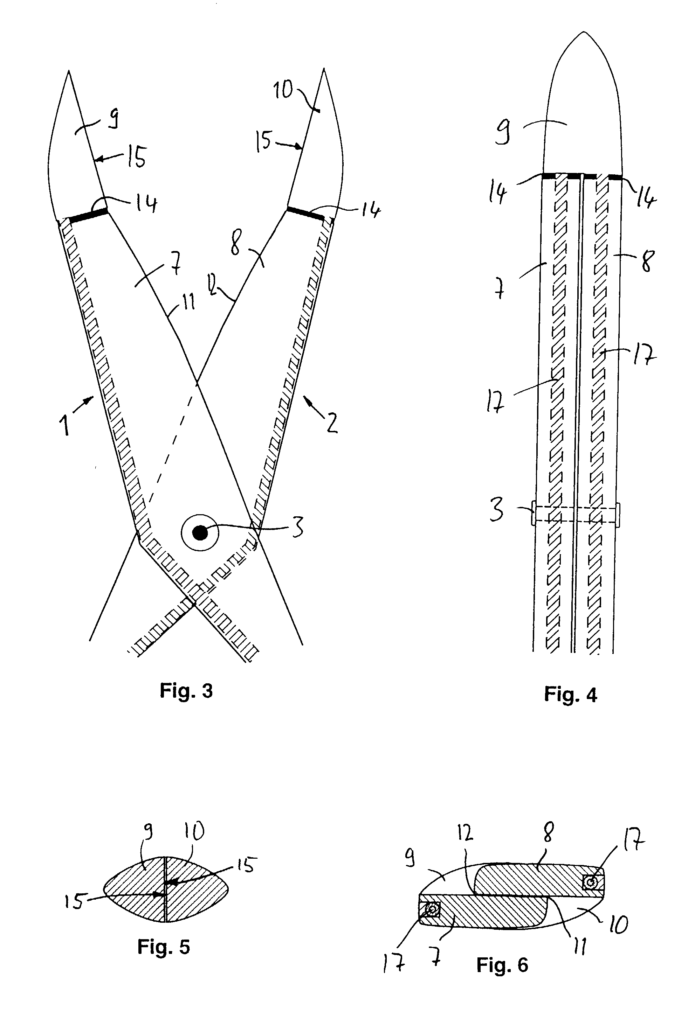

As shown in Figs. 2 and 6, two electrically insulated wires 17 extend from the electrode sections 9, 10 through grooves 18 in the outer edges of the scissor members 1, 2 to the grips 4, 5, from where they are connected to a power supply. The grooves 18 are filled with a heat resistant resin or metal filling.

The scissors are used as follows: For cauterising, the tissue is pinched between the electrode sections 9, 10 and voltage is applied to the wires 17. For cutting, no voltage is applied and the blade sections 7, 8 are used for shearing the tissue.

Since cauterising takes place at the distal end of the scissors and shearing in a more proximal section, consecutive steps of cauterisation with subsequent severing of tissue are carried out easily.

In the embodiment shown in the figures, both electrode sections 9, 10 have been electrically insulated from the blade sections 7, 8. If an asymmetric power supply is used, however, one of the electrodes can be grounded, in which case only one of the electrodes has to be electrically insulated and to be provided with a wire 17 through the scissor handle - the other electrode can be electrically connected to the second scissor handle, which is then connected to ground.

Figs. 2 and 5 show that the cauterising surfaces 15 are perpendicular to the pivoting directions 16. It is also possible to arrange the cauterising surfaces under an angle of up to 90° to the pivoting directions 16. However, an orientation substantially perpendicular to the pivoting directions 16 is preferred. In this context and in the claims, "substantially perpendicular" relates to all orientations where the tissue is primarily compressed but not sheared upon closing the scissors.

## Claims

1. Bipolar electrosurgical scissors comprising two pivotally connected scissor members (1, 2) with electrodes, characterized in that each scissor member (1, 2) is divided into a blade section (7, 8) for cutting tissue and an electrode section (9, 10) wherein said electrode section (9, 10) is arranged at a distal end of said blade section (7, 8).

2. The scissors of claim 1 wherein said electrode sections (9, 10) form a distal end (6) of said scissors.

3. The scissors of one of the preceding claims, wherein each electrode section (9, 10) comprises a cauterising surface (15) substantially perpendicular to a direction (16) of movement of said scissor members (1, 2).

4. The scissors of claim 3 wherein, in a closed position of said scissors, the cauterising surfaces (15) of said electrode sections (9, 10) are adjacent to and facing each other.

5. The scissors of claims 3 or 4 wherein, in a closed position of said scissors, said cauterising surfaces (15) are extending in a longitudinal direction of said scissors.

6. The scissors of one of the preceding claims, wherein, in a direction parallel to a pivot axis of said scissor members, at least one of said electrode sections (9, 10) is thicker than said blade sections (7, 8).

7. The scissors of one of the preceding claims, wherein said electrode sections (9, 10) are forming a forceps for squeezing tissue to be cauterised.

8. The scissors of one of the preceding claims, wherein at least one of said electrode sections (9, 10) is electrically insulated from the corresponding blade section (7, 8) and, in particular, wherein both of said electrode sections (9, 10) are electrically insulated from said blade sections (7, 8).

9. The scissors of claim 8 wherein at least one of said blade sections (7, 8) comprises an insulated conductor (17) for carrying a current to the corresponding electrode section (9, 10).

10. The scissors of one of the preceding claims, further comprising a pivot pin (3) connecting said scissor members (1, 2), wherein said blade sections (7, 8) substantially extend from said pivot pin (3) to said electrode sections (9, 10) and said electrode sections substantially extend from said blade sections (7, 8) to a distal tip (6) of said scissors.
